# EUROPEAN PATENT SPECIFICATION

(11) **EP 1 596 862 B1**
(45) Date of publication and mention of the grant of the patent: **08.12.2010**
(21) Application number: 04712041.5
(22) Date of filing: 18.02.2004
(51) Int. Cl.: A61K 31/4748, A61P 1/08

(54) **USE OF PALONOSETRON FOR TREATING POST-OPERATIVE NAUSEA AND VOMITING**
VERWENDUNG VON PALONOSETRON ZUR BEHANDLUNG VON POSTOPERATIVER ÜBELKEIT UND ERBRECHEN
UTILISATION DE PALONOSETRON POUR TRAITER LA NAUSEE ET LES VOMISSEMENTS POSTOPERATOIRES

(30) Priority: 18.02.2003 US 448342 P
(43) Date of publication of application: 23.11.2005
(60) Divisional application: 10177400.8
(73) Proprietor: Helsinn Healthcare S.A., 6915 Pambio-Noranco (CH)
(72) Inventor: BARONI, Luigi, 22060 Bregnano, Como (IT); MACCIOCCHI, Alberto, deceased (CH); BRAGLIA, Enrico, CH-6912 Pazzallo (CH); BRAGLIA, Riccardo, CH-6912 Pazzallo (CH)
(74) Representative: Zardi, Marco
(86) International application number: PCT/EP2004/001558
(87) International publication number: WO 2004/073714

(56) References cited:
- WO-A-03/100091
- WO-A1-2004/067005
- US-A1- 2003 095 926
- TANG, JUN ET AL: "The efficacy of RS-25259, a long-acting selective 5-HT3 receptor antagonist, for preventing postoperative nausea and vomiting after hysterectomy procedures" ANESTHESIA & ANALGESIA (BALTIMORE) (1998), 87(2), 462-467 , XP009027884
- CHELLY J ET AL: "Oral RS-25259 prevents postoperative nausea and vomiting following laparoscopic surgery" ANESTHESIOLOGY (HAGERSTOWN), vol. 85, no. 3A, 1996, page A21 XP008030467 Annual Meeting of the American Society of Anesthesiologists;New Orleans, Louisiana, USA; October 19-23, 1996 ISSN: 0003-3022
- TANG J ET AL: "Efficacy of RS-25259, a novel 5-HT-3 antagonist, in the prevention of postoperative nausea and vomiting after major gynecologic surgery" ANESTHESIOLOGY (HAGERSTOWN), vol. 87, no. 3 SUPPL., 1997, page A329 XP008030479 Annual Meeting of the American Society of Anesthesiologists;San Diego, California, USA; October 18-22, 1997 ISSN: 0003-3022
- STACHER, GEORG: "Palonosetron Helsinn" CURRENT OPINION IN INVESTIGATIONAL DRUGS (PHARMAPRESS LTD.) (2002), 3(10) 1502-1507, XP009027887
- NAVARI, RUDOLPH M.: "Pathogenesis-based treatment of chemotherapy-induced nausea and vomiting-two new agents" JOURNAL OF SUPPORTIVE ONCOLOGY (2003), 1(2), 89-103 , XP001189244
- EGLEN R M ET AL: "PHARMACOLOGICAL CHARACTERIZATION OF RS 25259-197, A NOVEL AND SELECTIVE 5-HT3 RECEPTOR ANTAGONIST, IN VIVO" BRITISH JOURNAL OF PHARMACOLOGY, NATURE PUBLISHING GROUP, BASINGSTOKE, HANTS; GB, vol. 114, no. 4, 1 January 1995 (1995-01-01), pages 860-866, XP009027881 ISSN: 0007-1188
- GASTER ET AL: MEDICINAL SEARCH REVIEWS,, vol. 17, no. 2, 1997, pages 163-214,

## Description

### FIELD OF THE INVENTION

The present invention discloses medical uses to treat post-operative nausea and vomiting, as well as emesis generally, with 5-HT₃ receptor antagonists. In particular, the invention discloses medical uses for reducing post-operative nausea and vomiting and other emetic events with palonosetron.

### BACKGROUND OF THE INVENTION

Post-operative nausea and vomiting (PONV) is one the most common consequences of many anesthetic and surgical procedures. PONV can cause serious side effects such as dehydration, electrolyte imbalance, gastric hemiation, wound disruption, esophageal tears, and muscular fatigue. Aside from the medical complications, PONV can cause patients to experience anxiety about undergoing further surgery. Because of delayed recovery and discharge, as well as enhanced medical care, PONV adds substantial costs to an already burdened health care system.

Typically, PONV occurs within 24 hours of surgery. At least between 25 and 40% of surgical patients have PONV. PONV has been correlated with age, obesity, type and duration of surgery, duration of general anesthesia, amount of visceral manipulation, early ambulation, use of opiate analgesics postoperative, and pain. The incidence of PONV averages between 20 and 30% across surgery types (Watcha, M.F. and White, P.F. (1992) "Post-Operative narusa nad vomiting: its etiology, treatment and prevention." Anesthesiology, 77:162-184), however five general types of surgical procedures (those of the ear, nose, throat; eye; gynecological; gastrointestinal; and cardiovascular) account for Approximately 69% of all PONV cases.

No single drug or class of drug is fully effective in controlling PONV. It has been suggested that prophylactic anti-emetic therapy can be more cost-effective compared with treatment of established symptoms where operations are associated with a high risk of emesis (Watcha MF and Smith I. (1994) "Cost-effectiveness analysis of anti-emetic therapy for ambulatory surgery." J Clin Anesth, 6: 370-7).

The number of operations done per year in the Western world and Japan is on the order of 65 million. Many anesthetists and anesosiologists currently use prophylactic anti-emetics such as low dose metoclopramide (10 mg) pre- or peri-operatively and many use no prophylactic anti-emetics at all due to poor efficacy of current agents coupled with troublesome side-effects such as dystonic reactions and somnolence. Thus the need for a safer and efficacious antiemetic in PONV is present.

PONV is thought to be mediated through serotonin (5 hydroxytryptamine, 5HT) receptors in the gastrointestingal tract, the nucleus solitarius, and the chemoreceptor trigger zone of the area postrema. Contributing factors include the release of 5HT from enterochromaffin cells in the small bowel, which leads to the stimulation of vagal afferent fibers that in turn cause 5HT release in the area postrema and direct chemoreceptor trigger zone stimulation by opiates and anesthetic drugs. Although vestibular stimulation is not mediated through 5HT3 receptors, when it is enhanced by the effects of residual anesthetic drugs it also contributes to PONV.

There are nine groups of agents that are used clinically for the treatment of emesis, including: anticholinergics (e.g. scopolamine), antihistamines (e.g. hydroxyzine, promethazine), phenothiazines, butyrophenones (e.g. droperidol), cannabinoids, benzamides, glucocorticoids, and benzodiazepines (*Merck Manual* (1992) Merck Research Laboratories).

In recent years an additional class of drugs referred to as 5-HT₃ (5-hydroxytryptamine) receptor antagonists has been developed that treat emesis by antagonizing cerebral functions associated with the 5-HT₃ receptor. See "Drugs Acting on 5-Hydroxytryptamine Receptors" (Sep. 23, 1989) *The Lancet* and refs. cited therein. Competitive antagonists of the 5-HT3 receptor are used clinically as antiemetic agents. These agents include: ondansetron (Zofran, Glaxo Welcome), granisetron (Kytril, SmithKline Beecham) and tropisetron (Navoban, Sandoz), and dolasetron (Anzemet, Aventis). At the present time, the 5-HT3 competitive antagonists in combination with the corticosteroid dexamethasone represent the best prophylaxis and treatment of acute nausea and/or vomiting.

U.S. Patent No. 4,695,578, to Glaxo discloses 1,2,3,9-tetrahydro-3-imidazol-1-ylmethyl-4H-carbazol-4-ones compositions (ondansetron, Zofran), potent selective antagonists of 5-HT receptors, useful in the treatment of migraine and psychotic disorders such as schizophrenia, while U.S. Patent Nos. 4,753,789, 4,929,632, 5,240,954, 5,578,628, 5,578,632, 5,922,749, 5,955,488, and 6,063,802, also to Glaxo, claims these compounds for the relief of nausea and vomiting. Zofran has been approved by the FDA for PONV, however, negative side effects have been noted in postoperative clinical tests. The most common are dizziness, headache, drowsiness, sedation, and constipation. Other adverse effects are diarrhea, dry mouth, and skin rash. Furthermore, the serum half life of ondansetron is on the order of 5.5 hours (Physicians desk reference (2002)), therefore treatment of PONV often requires multiple doses. In addition, the dose level can vary widely, depending on physician involvement Furthermore, it is suggested that Zofran be administered in three & mg doses or as a slow intravenous injection (Dupeyron, et al. (1993) "The effects of oral ondansetron in the prevention of postoperative nausea and vomiting after major gynaecological surgery performed under general anesthesia" Anaesthesia v48, p214-18 (1993); Sung et al. "A double blind, placebo controlled pilot study examining the effectiveness of intravenous ondansetron in the prevention of postoperative nausea and ernesis" J. Clin Anesth v5, p22-29 (1993); McKenzie et al. "A, randomized, double blind pilot study examining the use of intravenous ondansetron in the prevention of postoperative nausea and vomiting in female inpatients" J. Clin. Anesth. V5, p30-36 (1993)).

Kytril (granisetron hydrochloride), a selective blocking agent of the 5-HT₃ receptor is currently used for both the prevention and treatment of PONV. U.S. Patent Nos. 5,952,340, 4,886,808, 4,937,247, 5,034,398 and 6,294,548, to SmithKLine Beecham, filed May 23, 1996 disclose a method of treatment of PONV by administration of granisetron to surgical patients. The patents state that granisetron can be administered intravenously, either pre-operatively, peri-operatively; or postoperatively. When used for prevention, Kytril is given just before or during surgery to prevent PONV from occurring. In the case of treatment, Kytril is given to a patient who experiences PONV after surgery is completed. However, Kytril can cause headache, constipation, weakness, drowsiness or diarrhea. The plasma half-life of Kytril is also found to be less than a day, ranging from 1.77-17.73 hours.

Dolasetron is used to prevent nausea and vomiting caused by cancer chemotherapy, anesthesia, or surgery. Uses of this compound are detailed in U.S. Patent Nos. 4,906,755 and 5,011,846, directed to a group of esters hexahydro-8-hydroxy-2,6-methano-2H-quinolizin-3(3H)-ones useful in the treatment disorders including drug-induced vomiting, stimulation of gastric motility. Additionally, dolasetron serum half life is only approximately 7.5 hours (Physicians Desk Reference (2002)), therefore treatment of PONV could require administration of multiple doses. This is particularly disadvantageous as it can delay discharge of patients and thereby increase patient and insurance costs.

U.S. Patent Nos. 5,627,190, 5,430,040, 5,280,029, 5,137,893, to G. D. Searle & Co., describe imidazopyridines containing meso-azacycle side chains, which act as antagonists of the serotonin 5-HT₃ receptor and can be useful for the treatment of emesis.

Additional 5HT receptor antagonists have been found. For example, Ponchant et al. (1991) "Synthesis of 5-125I-Iodo-Zacopride, A New Probe for 5-HT3 Receptor Sites," J. Lab. Cpds. and Radiopharm., Vol. XXIX, No, 10, pp. 1147-1155, discloses substituted 3-quinuclidinyl benzamides useful for 5-HT₃ serotonin receptor binding. U.S. Patent Nos. 4,707,484, 5,189,041, 5,202,333, 5,491,148, and 5,492,914 to Syntex, disclose a class of benz[*de*]isoquinolin-1-ones that act as 5-HT₃ receptor antagonists. Species disclosed in the '333 patent include palonosetron. According to this patent, the class of compounds is useful to treat emesis, including emesis from surgical anesthesia, gastrointestinal disorders, anxiety, depressive states, and pain. In addition, the '333 patent discloses a general dose range for administration that may range from .1ng/kg to 1 mg/kg body weight per day, preferably from 10 to 100,000 ng/kg/day. However, no enabling examples are provided to allow administration of these species for PONV. The Syntex patents do not disclose any specific data for determining a suitable therapeutic regimen such as the potency of the compounds, the serum half life of the compounds, dose response data, or duration of effect.

The introduction of 5-HT₃ receptor competitive antagonists into clinical practice revolutionized the treatment of emesis because these agents are more efficacious and have fewer side-effects than antiemetic agents from the other groups (Markham, A. & Sorkin, E. M. (1993) Drugs, 45:931-952). The American Society of Anesthesiologists suggest 5HT₃ antagonists as the standard treatment and preventative therapy for PONV, used in 74-78% of patients. Each of the currently available 5-HT₃ antagonists suffers from one or more of the following deficiencies which limits its therapeutic utility: potency, duration of effect, window of therapeutic efficacy, ease of dosing, side effects, and certainty of the dosing regimen. Sabra, K (1996) id. Although side effects are typically mild to moderate and transient, they include headache, lightheadedness or dizziness, abdominal pain or cramping, constipation, sedation and fatigue, elevations in hepatic transaminases and/or bilirubin, and electrocardiographic changes. (Gregory , RE and Ettinger, DS (1998) "5HT3 receptor antagonists for the prevention of chemotherapy-induced nausea and vomiting. A comparison of their pharmacology and clinical efficacy" Drugs, 55(2):173-189.) In addition, these drugs are limited in their efficacy. Specifically, 38-50% of patients require additional therapy after treatment with the most popular 5-HT₃ antagonist, Zofran (Phillip Scuderi et al. (2002) "Single-Dose Oral Ondansetron Prevents Nausea and Vomiting After Inpatient Surgery" Applied Research, 1(1)). This is in part due to the short half life and relatively low potency of the drug. The serum half-life of Zofran is approximately 5-6 hours, and typically 3-4 doses must be given to effectively overcome PONV.

5-HT₃ receptor competitive antagonists are currently also very expensive. Consequently, they are not prescribed to the extent that they are needed and the cost of these agents is a burden to the health care system.

One of the greatest challenges in drug dosing is to find a dose that is well-tolerated and consistently efficacious. Finding an optimum dose is complicated by such factors as serum half-life, dosing/efficacy relationships, and, in the case of PONV, the variables inherent in different operative procedures, different anesthetics used, and post-operative treatments required. This challenge is particularly acute when devising single unit dose formulations of the anti-emetic drug that is efficacious over a range of body weights, because single unit dose forms are designed typically to prevent nurses and doctors from titrating the dose in the clinic.

### OBJECTS OF THE INVENTION.

It is an object of the present invention to inhibit PONV using 5-HT₃ receptor antagonists that have improved potency.

It is an object of the present invention to inhibit PONV using 5-HT₃ receptor antagonists that can be administered at doses that yield fewer incidences of unwanted side effects.

It is a further object of the present invention to inhibit PONV using 5-HT₃ receptor antagonists that can be administered fewer times to reduce the occurrence of unwanted side effects.

It is a further object of the present invention to inhibit PONV using 5-HT₃ receptor antagonists that requires less rescue therapy, potentially reducing the incidence of side effects.

Another object of the present invention is to provide defined doses of palonosetron that can be administered to a patient of nearly any body weight to control PONV.

Still another object of the present invention is to provide 5-HT₃ antagonists that possess increased plasma half-life and prolonged *in vivo* activity.

Another object of the invention is to provide greater flexibility when administering emesis-inhibiting agents before an operation by increasing the size of the window for pretreatment.

Yet another object of the invention is to provide a PONV reducing agent which can be administered orally.

It is a further object of the present invention to provide a PONV reducing agent that can be administered intravenously.

Yet another object of the invention is to provide a PONV reducing agent which can be administered before or after an operation.

Yet.another object of the invention is to reduce the cost of PONV therapy.

Still further objects of the invention pertain to the use of palonosetron in the general prevention or treatment of emesis, regardless of the cause.

### SUMMARY OF INVENTION

It has surprisingly been discovered that a remarkably small dose of palonosetron is effective against PONV, and that such a small dose is effective for practically the entire time that a patient undergoing a surgical procedure is usually at risk for developing PONV (typically within about 0-36 hours of the operation).

Object of the invention is the use of from 0.025 to 0.250 mg of a treatment-effective amount of palonosetron in the manufacture of a medicament for the treatment or prevention of post-operative nausea and vomiting (PONV) in a human, wherein said palonosetron is contained in a liquid formulation at a concentration ranging from 0.03 to 0.2 mg/ml, and wherein said palonosetron is administered in a window about 4 hours before the operation to about 4 hours after the operation.

The above use is effective against PONV caused by a variety of surgical procedures and anesthetic compounds, as discussed in greater detail below.

A feature of the invention is the unexpectedly long duration of action of palonosetron, and the ability of the compound to prevent "delayed-onset" PONV (i.e. PONV occurring greater than about 4, 6, 8, 12, or 18 hours after surgery):

Another feature derives from the unexpectedly rapid onset of palonosetron following admimstration, and its use as a rescue medication when PONV is unexpectedly experienced.

The surprising potency and the extended plasma half-life of palonosetron also satisfy a number of unmet clinical needs including: increased efficacy, decreased cost, decreased side effects and increased duration of action.

The emesis can be induced by any number of medical procedures, including chemotherapy, radiotherapy, and surgical operations.

Palonosetron has also surprisingly been found to exhibit an efficacy plateau, which when combined with its safety profile allows for a single dose to be effectively administered across a range of body weights.

### DETAILED DESCRIPTION

### Definitions

"Vial" means a small glass container sealed with the most suitable stopper and seal, other suitable primary containers may be used, for instance, but not limited to, pre-filled syringes. Vial also means a sealed container of medication that is used one time only, and includes breakable and non-breakable glass vials, breakable plastic vials, miniature screw-top jars, and any other type of container of a size capable of holding only one unit dose of palonosetron (typically about 5 mls.).

Throughout this specification the word. "comprise," or variations such as "comprises" or "comprising," will be understood to imply the inclusion of a stated element, integer or step, or group of elements, integers or steps, but not the exclusion of any other element, integer or step, or group of elements, integers or steps

"PONV" includes any episodes of emesis in the 4 day period following an operation.

"Operation" is any medical procedure during which surgery is performed or general anesthetics are administered to a patient.

"Emesis", for the purposes of this application, will have a meaning that is broader than the normal, dictionary definition and includes not only vomiting, but also nausea and retching.

"Palonosetron" means (3aS)-2,3,3a,4,5,6-Hexahydro-2-[(S)-1-Azabicyclo[2.2.2]oct-3-yl]2,3,3a,4,5,6-hexahydro-1-oxo-1*H*benz[*de*]isoquinoline, and is, in one embodiment, present as the monohydrochloride. Palonosetron monohydrochloride can be represented by the following chemical structure:

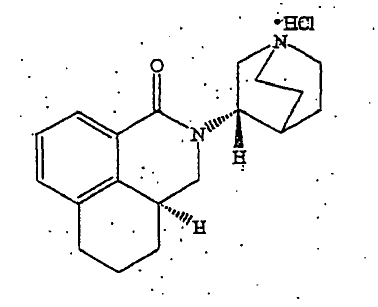

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Pharmaceutically acceptable salts" means salts which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, o-(4-hydroxybenzoyl)benzoic acid, cinnamic acid, mandelic acid, methanesulfonic acid, ethanesulfonic acid, 1,2,-ethanedisulfonic acid, 2-hydroxyethanesulfonic acid, benzenesulfonic acid p-chlorobenzenesulfonic acid, 2-naphthalenesulfonic acid, p-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like.

In addition, pharmaceutically acceptable salts may be formed when an acidic proton present is capable of reacting with inorganic or organic, bases. Acceptable inorganic bases include sodium hydroxide, sodium carbonate, potassium hydroxide" aluminum hydroxide and calcium hydroxide. Acceptable organic bases include ethanolamine, diethanolamine, triethanolamine, tromethamine, N-methylglucamine and the like.

### Methods of Administration

One embodiment of the present invention is premised upon the discovery that palonosetron is surprisingly more potent than other 5-HT₃ antagonists in its ability to treat emesis due to an operation. This emesis may be caused either by a surgical procedure or be due to the administration of an anesthetic compound during such an operative procedure.

The present treading or preventing PONV in a human comprises admmistering from about 0.025 to about 025 mg of a treatment-effective amount of palonosetron or a pharmaceutically acceptable salt thereof substantially at the time of an operation. In one embodiment, the drug is administered substantially at the time of the operation (i.e. in a window of within about 1, 1.5, 2 or 4 hours before the operation, to within about 1, 1.5, 2 or 4 hours after the operation, or during the operation). Administration during an operation can occur, for example, intravenously after skin closure but before leaving the operating room.

The method can be performed in a single administration of the drug, and can be unaccompanied by further administrations of palonosetron, palonosetron derivatives, or other rescue medications during the period in which PONV from an operation is a risk.

Another embodiment derives from the unexpectedly long duration of action of palonosetron, and the ability of the compound to prevent delayed-onset PONV (i.e. PONV occurring greater than about 4, 6, 8, 12, or 18 hours after surgery, but typically within about 36 hours of surgery). Thus, in another embodiment the invention provides treating or preventing delayed-onset post-operative nausea and vomiting (PONV) comprising administering a treatment effective amount of palonosetron or a pharmaceutically acceptable salt thereof substantially at the time of an operation.

Based upon the surprising efficacy plateau and safety profile of palonosetron, methods have been developed in which a single dose is administered across a range of body weights. Thus, in yet another embodiment the invention provides a method of treating or preventing PDNV comprising administering a defined dose of palonosetron or a pharmaceutically acceptable salt thereof to a plurality of patients ranging in weight from about 50 to about 80 kilograms, wherein said defined dose is the same among said plurality of patients. A plurality means more than one. It will be understood, however, that a doctor may treat more than 5 or even 10 patients during a busy period of time, and that the number of patients (and doses of palonosetron administered) will vary from doctor to doctor. Said period of time is not important to the invention, but may be one day, seven days, or even 30 days during which said doctor may treat greater than 5, 10 or even 20 patients with a single dose of palonosetron.

### Dosing

For PONV, doses ranging from about 0.2 to about 30 µg/kg from about 03 to about 10 µg/kg, or from about 0.3 to about 1.0 µg/kg, are generally suitable as are specific doses of about 0.3, 0.65 and 1.0 µg/kg. A particularly effective dose for PONV regardless of the patient's body weight has been found to range from about 0.025 to about 0.075 mg of palonosetron. Specfic doses with which the invention can be practiced against PONV include about 0.025, 0.050, 0.075, 0.1, 0.2, and 0.25 mg of palonosetron.

It has surprisingly been discovered that the clinical response substantially plateaus at about 1 microgram/ kilogram of body weight Therefore, efficacy can be expected over a wide range of patient body weights when utilizing doses approximating this amount. These doses can be administered intravenously or orally, and when administered orally may be administered as a liquid, solid or soft gel capsule.

Another particular advantage associated with the lower dosages of palonosetron is the ability to administer the drug in a single intravenous bolus over a short, discrete time period. This time period generally extends from about 10 to about 60 seconds, or about 10 to about 40 seconds, or about 10 to 30 seconds.

The uses of the present invention can be practiced with mammals other than humans, employing substantially the same dosages when based upon the weight of the animal.

### Associated medications and procedures

The inventors have shown that palonosetron is effective against PONV initiated by a variety of surgical procedures. In addition, they have shown that palonosetron is effective against PONV induced by a number of anesthetic compounds.

Operations that can be performed to induce PONV can include any operation, including those with high likelihood of inducing PONV. These can include, but are not limited to surgical procedures including gynecologic procedures, including abdominal or vaginal hysterectomy; abdominal and gastromtestinal procedures and bowel manipulation. Procedures can also include laparoscopic surgical procedures, ear nose and throat procedures, and ophthalmic procedures.

The duration of an operation is a major factor in the development of PONV. Extended surgical procedures are more likely to lead to PONV than shorter operations. Compounds of slow onset must be given before an operation, to ensure that they are effective within a reasonable time after the operation. On the other hand, compounds with rapid onset typically show a short duration of action, and thus cannot be given to a patient before a lengthy procedure. Because it shows a rapid onset, palonosetron can be administered after surgical procedures of any length. At the same time, due to the extended half-life of palonosetron in the body and its limited side-effects, palonosetron, can also be administered before an operation, including operations that are time consuming. In particular, one advantage of palonosetron is that it can be administered before exploratory operations which may lead to lengthy procedures that cannot be forecast with accuracy. Thus, in one embodiment, palonosetron or a pharmaceutically acceptable salt or prodrug thereof is delivered before exploratory surgery. In a subembodiment, the drug is delivered about 1, 15, or 2 hours before surgery. In another embodiment, the drug is delivered less than one hour before surgery, for example 10, 20, 30, or 45 minutes before surgery.

Certain anesthetic agents have been associated with a higher incidence of PONV than others. Certain types of premedication, use of opioid analgesics, nitrous oxide, some inhalation agents, and greater depth of anaesthesia can all affect the incidence of PONV. For the purposes of this invention, anytype of anesthestic can be utilized. These include, but are not limited to thiopental, nitrous oxide, isoflurane, enflurane, fentanyl, sufentanil, morphine, meperidine, hydromorphone, or narcotic antagonists, vecuronium, succinylcholine, or tubocuarine alone or any combination thereof Anesthetics also include schedule II narcotics such as: opium, morphine, methadone and codeine, 1-Diphenyl-propane-carboxylic acid, 2-Methyl-3-morpholino-1, 4-Cyano-2-Dimethylamino-4, 4-Diphenyl butane, Alphaprodine HCl-(Nisentel), Anileridine, Benzitramide, Codeine, Dihydrocodeine, Diphenoxylate, Ethylmorphine, Etorphine Hydrochloride, Fentanyl, Granulated Opium, Hydrocodone, Hydromorphone (Dilaudid), Isomethadone, Levoalphacetylmethadol (LAMM), Levomethorphan, Levorphanol (Levo-Dromoran), Merperidine (Demeral, Pethadol), Metazocine, Methadone (Dolophine), Methadone Intermediate, Metapon, Moramide Intermediate, Morphine, Opium fluid, Opium Extracts, Opium Tincture, Oxycodone , Oxymorphone (Numorphan), Pantopon (Hydrochloride, opium alkaloids), Pethidine, Pethidine-Interlediate-A, 4 cyano-1-methyl-4-phenylpiperdine, Pethidine-Intermediate-B ethyl-4-phenylpiperdine-4-carboxylate, Pethidine-Intermediate-C 1-methyl-4-phenylpiperidine-carboxylic acid, Phenazocine, Piminodine, Poppy Straw, Powdered Opium, Racemethorphan, Racemorphan, Raw Opium, Remifentanil, Raw Opium Extracts, Thebaine. Additionally, combinations such as: Oxycodone & Acetaminophen tablets, Oxycodone HCl, Oxycodone Terephthalate & Aspirin tablets, Oxycodone with Acetaminophen, Oxycodone with Aspirin tablets, Percodan-Demi tablets, Percodan tablets, Tylox Capsules. Hallucinogenic substances such as: 1-Dronabinol (some other names for dronabinol: [6aR-trans]-6a,7,8, or (-) delta-9-[trans]-tetrahydrocannabinol 2-Nabilone (another name for Nabilone): [+]-trans-3-(1,1-deimethylheptyl)-6,6a,7,8,10,10a-hexahydro-1-hydroxy-6,6-dimethyl-9H-dibenzo [b,d]pyran-9-one. Opiates such as: Alfentanil, Bulk Dextropropoxyphene (non-dosage form), Carfentanil, Sufentanil. Stimulants such as: Adderall, Coca Leavens, Cocaine, Dextroamphetamine, Ecgonine, Methamphetamine, Methylphenidate, Phenmetrazine. Depressants such as: Amobarbita (Amytal), Amobarbital + Secobarbital (Tuinal), Glutethimide (Doredin), Pentobarbital (Nembutal), Secobarbital (Secorial), 1-Any drug approved by the United States Food and Drug Administration for marketing only as a suppository including Amobarbital, Pentobarbital or Secobarbital shall be in Schedule 11, 2-Immediate Precursors. A material, compound, mixture or preparation which contains a quantity of the following: immediate precursors to amphetamine and methamphetamine; Phenyl-2-propanone, P2P, Benzyl methyl ketoneMethyl benzyl ketone, immediate precursors to phencyclidine, 1-phenycyclohexylamine, 1-piperidineocyclohexaneecarbonitrile pcc. Additional administration of the schedule III drugs such as: Aspirin with Codeine, Codimal PH, Empirin with Codeine, Fioricet with Codeine, Fiorinal with Codeine, Hycodan tablets, Nalline, Nucofed, Nucofed Experctorant Syrup with Codeine, Phenaphen with Codeine, Talwin; Pentazocine, all forms including its salts, Tylenol with Codeine No. 1, 2, 3, and 4, Vanex-HD Liquid, Bancap, Codamine, Codiclear DH Syrup, Codimal PH Syrup, Co-Gesic tablets, Detussin, various, Duocet, Entuss D Liquid, Hitussin Ed Tuss HC liquid, Hycodan, Hycomine, Hycomine Pediatric Syrup, Hycotuss Expectorant, Hydrocodone Compound Syrup, Hydropane, Hydrophen, Hy-Phen Tablets, Lorcet, Lortab, Rolatuss with Hydrocodone, S. T. Forte, S. T. Forte Liquid 2, Triaminic Expectorant DH, Tussaminic DH Forte, Tussanil DH Syrup, Tussionex, Vanex-HD, Paragoric, Benzphetamine, Chlorphentermine, Chlortermine, Phendimetrazine to include but not necessarily be limited to: Adipost, Adipex-P, Anorex ,Bontril PDM, Melfiat, Melfiat - 105 Unicells, Metra, Obalan, Obezine, Parzinae, Phendiet, Phendiet 105, Plegine, Prelu-2, PT 105, Rexigen Forte, Wehless, Wehless-105 Timecells. In addition: Methamphetamine HCl, Conjugated Estrogen, Methyltestosterone, any material, compound, mixture, or preparation containing amobarbital, secobarbital, pentobarbital, or any of their salts and one or more active medicinal ingredients that is' not a controlled substance. Any suppository form that contains amobarbital, secobarbital or pentobarbital approved only for use in suppository form. Any substance which contains any quantity of a derivative of chlorhexadol, glutethimide, lysergic acid, lysergic acid amide, methylprylon, sulfondiethylmethane, sulfonethylmethane, sulfone methane. Tiletamine and zolazepam or any of their salts, Other names for tiletamine are: 2-(ethylamino)-2-(2-thienyl)-cyclohexanone, Other names for Zolazepam are: 4-(2-flurophenyl)-6,8-dihydro-1,3,8-trimethylpyrazolo-(3,4-e)(1,4)diazepin-7(1H)-one, flupyrazpon, Butabarbital-Butisol, Chloral Hydrate, Mephobarbital, Metharbital, Methytprylon, Phenobaribtal, Sulfomethane, Sulfondiethylmethane, Sulfonethylmethane, and Talbutal.

It has also been found that the duration in which a patient is anesthetized is correlated to the likelihood that the patient will experience PONV. Thus, in various embodiments, anesthesia is administered to the patient for 1-12 hours, 1-8 hours, 1-6 hours, or 1-4 hours.

In another embodiment the palonosetron is administered in the presence or absence of a steroid such as dexamethasone.

### Pharmaceutical Compositions

Although the foregoing discussion has focused upon intravenous and oral administration of palonosetron or pharmaceutically acceptable salts or prodrugs thereof, the methods of the present invention can be performed by administering the active material by any appropriate route, for example, orally, parenterally, intravenously, intradermally, intramuscularly, transdermally, intranasally, subcutaneously or topically, by suppository in liquid or solid form.

A particularly surprising advantage of the lower dosages required of palonosetron derives from the fact that the stability of palonosetron increases in solution as its concentration decreases. The potency of palonosetron thus allows the palonosetron to be formulated in stable compositions comprising a. wide range of concentrations. The composition is a liquid formulation in which the palonosetron is present as a concentration of from about 0.03 to about 0.2 mg/ml. In one particular embodiment the palonosetron is present in a liquid formulation at a concentration of about 0.05 mg/ml.

The pH of oral solutions of palonosetron suitably ranges from about 4 to about 6, and may suitably be about 5.

In one particular embodiment the palonosetron is supplied in a single unit dose vial, such as for intravenous administration, comprising from about 0.1 to about 10.0 ml, or about 0.5, 1.0 or 1.5 ml, of solution. In one embodiment, the vial includes about 0.025, 0.05, 0.075 or 0.1 mg of palonosetron at a concentration of about 0.05 mg/ml.

The enhanced stability allows the palonosetron to be stored for extended periods of time, exceeding about 1 month, 3 months, 6 months, 1 year, 18 months, or 2 years, but preferably not extending beyond 36 months, This enhanced stability is seen in a variety of storage conditions, including at room temperature.

The compound can be administered as a component of an elixir, suspension, syrup, the like. A syrup may contain, in addition to the active compounds, sucrose as a sweetening agent and certain preservatives, dyes and colorings and flavors.

Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. If administered intravenously, preferred carriers are physiological saline or phosphate buffered saline (PBS).

### EXAMPLES

### EXAMPLE 1. PREVENTION OF PONV USING A SINGLE INTRAVENOUS DOSE OF PALONOSETRON

A study was conducted to test the efficacy and safety of five doses of intravenously administered palonosetron for the prophylaxis of postoperative vomiting and nausea. A total of 381. women, 24-80 years of age, scheduled for abdominal or vaginal hysterectomy and having ASA physical status rating of I or II were enrolled in this study. All patients received balanced general anesthesia, including N₂O plus an opiate. Study medication, palonosetron, was administered over 30 seconds through a peripheral IV line at skin closure at the conclusion of the surgical procedure.

**Table 1: 24 Hours After Recovery - IV (Evaluable patients)**

| | Placebo o (n=62) | 0.1 µg/kg (n=47) | 0.3 (n=67) µg/kg | 1.0 µg/kg (n=62) | 3.0 (n=67) µg/kg | 30 µg/kg (n=67) |
|---|---|---|---|---|---|---|
| % with CR* | 19% | 34% | 34% | 44% | 30% | 45% |
| p.Value^{a} | - | N/A^{b} | 0.051 | 0.004 | 0.174 | 0.002 |
| % with CC** ^{a} | 19% | 34% | 33% | 44% | 30% | 45% |
| p-Value^{a} | - | N/A^{b} | 0.075 | 0.004 | 0.174 | 0.002 |
| Time to Failure (hr). (median)*** | 3.3 | 6.0 | 9.6 | 19.5 | 7.3 | 15.0 |
| p-Value^{a} | - | N/A^{b} | 0.005 | < 0.001 | 0.040 | 0.002 |
| % no EE§ p-value^{a} | 47% | 55% | 60% 0.137 | 73% 0.003' | 61% 0.101 | 76% < 0.001 |

| | | | | | | |
|---|---|---|---|---|---|---|
| * Complete response (CR): The proportion of patients who completed the 24-hour postrecovery observation period without experiencing an emetic episode and without receiving rescue antiemetic therapy. ** Complete control (CC): The proportion of patients who had a complete response and only mild or no nausea *** Median time (hours) to treatment failure (time to failure): Either emesis or administration of rescue medication; whichever occured first § no emetic episode, no rescue therapy ^{a} p-value for treatment effect versus placebo; ^{b}N/A = not analyzed | | | | | | |

### Onset and Duration of Antiemetic Activity

Within the first 4 hours of recovery, between 66% and 72% of the evaluable patients treated with palonosetron 0.3-30 µg/kg had a complete response, compared with 48% of placebo-treated patients (30/62). The 0.3-, 1-, and 30-µg/kg dose groups were statistically significantly superior to placebo (p = 0.008-0.035), whereas the 3-µg/kg dose group was nearly significantly different (p 0.059). In addition, 57% of the 0.1-µg/kg-treated patients (27/47) had a complete response.

Over this same time period, statistically significantly more patients in the 0.3-, 1-, and 30-µg/kg treatment groups were free from emetic episodes compared with the placebo group (90-94% vs. 73%; p = < 0.001-0.015), whereas there was no difference between the 0.1- and 3-µg/kg dose groups versus placebo.

| **Table 2: 4 Hours After Recovery - IV** | | | | | | |
|---|---|---|---|---|---|---|
| | Placebo (n = 62) | 0.1µg/kg (n = 47) | 0.3µg/kg (n = 67) | 1 µg/kg (n = 62) | 3 µg/kg (n = 67) | 30 µg/kg (n = 67) |
| % with CR p-value^{a} | 48% | 57% | 72% 0.008 | 69% 0.023 | 66% 0.059 | 66% 0.035 |
| % no EE p-value^{a} | 73% | 68% | 90% 0.015 | 92% 0.005 | 78% 0,568 | 94% < 0.001 |

| | | | | | | |
|---|---|---|---|---|---|---|
| ^{a} p-Value for treatment effect versus placebo | | | | | | |

Two hours postrecovery, 24.2% of placebo-treated patients (15/62) experienced an emetic episode, compared with 4.5% (3/67), 6.5% (4/62), and 10.4% (7/67) of patients in the 30-µg/kg, 1-µg/kg, and 0.3-µg/kg treatment groups respectively. At 12 hours, 13.4% (9/67), 14.5% (9/62), 29.9% (20/67), end 45.2% (28/62) of 30-µg/kg--, 1-µg/kg-, 0.3-µg/kg-, and placebo-treated patients experienced one or more emetic episodes.

### Rescue Medication

The "failure rate" for patients experiencing emetic episodes between Hours 12 and 24 decreased relative to the first 12 post-recovery hours. At 24 hours, 23.9% (16/67), 27.4% (17/62), 40.3% (27/67), and 53.2% (33/62) of 30-µg/kg-, 1-µg/kg-, 0.3-µg/kg-, and placebo-treated patients had experienced an emetic episode. Approximately 40% of evaluable placebo-treated patients (25/62, 40.3%) received rescue therapy within the first 2 hours, compared with approximately 21-31% of palonosetron-treated patients. At 8 hours, 62.9% of placebo-treated patients (39/62) received rescue medication, compared with 35.5-50.7% of palonosetron-treated patients. By 24 hours, more than three-quarters of placebo-treated patients (48/62, 77.4%) had received rescue therapy, compared with 50.7% (34/67) and 51.6% (32762) of palonosetron 30-µg/kg- and 1 µg/kg-treated patients, respectively, and 59.6% (28/47) and 59.7% (40/67) of palonosetron 0.1-µg/kg- and 0.3-µg/kg-treated patients, respectively.

| **Table 3: Duration of Effect (hours) - IV** | | | | | | |
|---|---|---|---|---|---|---|
| | Placebo (n = 62) | 0.1µg/kg (n = 47) | 0.3µg/kg (n = 67) | 1 µg/kg (n = 62) | 3 µg/kg (n = 67) | 30 µg/kg (n = 67) |
| Emetic Episode (25^{th} Percentile) | 2.3 16.3 | 2.2 > 24 | 8.4 > 24 | 21.8 > 24 | 4.9 > 24 | > 24 > 24 |
| Rescue (50^{th} percentile) | 4.3 | 8.5 | 9.9 | 21.3 | 8.0 | 23.2 |
| Time to Failure (50^{th} percentile) | 3.3 | 6.0 | 9.6 | 19.5 | 7.3 | 15.0 |

### Control of Nausea

Evaluable patients who received palonosetron 1 µg/kg, 3 µg/kg, and 30 µg/kg reported statistically significantly less nausea over the 24-hour period following recovery than those who received placebo. Nearly half of patients (32/67, 47:8%) who received 30 µg/kg reported no nausea, and no patients reported severe nausea. By comparison, 11.7% of placebo-treated patients (7/60) reported severe nausea and only 23.3% (14/60) reported no nausea (p-value for 30 µg/kg vs. placebo = 0.001). Only 2 patients each in the 1 µg/kg (2/61, 3.3%) and 3 µg/kg groups (2/66, 3.0%) reported severe' nausea, whereas 36.1% (22/61) and 34.8% (23/66) reported no nausea, respectively (p-value for 1 µg/kg vs. placebo = 0.009; p-value for 3 µg/kg vs. placebo = 0.049).

| **Table 4: Nausea During 24 Hours After Recovery - IV** | | | | |
|---|---|---|---|---|
| | Placebo (n = 62) | 1 µg/kg (n = 62) | 3 µg/kg (n = 67) | 30 µg/kg (n = 67) |
| No Nausea | 23.3% | 36.1% | 34.8% | 47.8 |
| Severe Nausea | 11/7% | 3.3% | 3.0% | 0.0% |
| Mean Score | 1.22 | 0.82 | 0.92 | 0.66 |
| p-value vs. Placebo | - | 0.009 | 0.049 | 0.001 |

### EXAMPLE 2. ORAL ADMINISTRATION OF PALONOSETRON TO TREAT PONV

A study was performed to test the efficacy, safety, and pharmacokinetics of five doses of orally administered palonosetron for the prophylaxis of postoperative vomiting and nausea. Three hundred fifty-one patients, 308 women and 43 men, 19-75 years of age, scheduled for elective laparoscopic surgical procedures and having an ASA physical status rating of I and II were enrolled in this study. All patients received balanced general anesthesia, including NO2 plus an opiate.

Doses of palonosetron were calculated based on the patient's body weight at the screening visit, rounded to the nearest kilogram. Each dose was diluted to a total volume of 25 mL by adding sterile water to the dosing cup. The dosing cup was rinsed with 25 mL of sterile water and the patient also swallowed this 25 mL. Study medication was administered 2 hours prior to scheduled induction of anesthesia for surgeries requiring nasogastric suction and 1 hour prior to scheduled induction of anesthesia for all other laparoscopic surgical procedures.

Orally-administered palonosetron reached a plateau with respect to clinical response at a dose of 1 µg/kg. At this dose, it was generally more effective than placebo and as effective as the higher doses of palonosetron.

### Twenty-Four-Hour Result

Statistically significant differences between palonosetron and placebo were observed at three dose levels of palonosetron (1, 10, and 30 µg/kg) and a near-significant difference for 3.0-µg/kg-treated patients (p = 0.059) with respect to the primary efficacy variable, the proportion of patients with a complete response, that is, the proportion of patients who completed the 24-hour postrecovery observation period without experiencing an emetic episode and without receiving rescue antiemetic therapy. At the 1, 10, and 30 µg/kg dose levels, 58%, 59%; and 53% of the patients, respectively, treated with palonosetron (p < 0.018) had a complete response as compared with 33% of placebo-treated patients. The more conservative intent-to-treat analysis of the complete response variable also demonstrated statistically significant differences for patients treated with 1, 3, 10, and 30 µg/kg.

| **Table 5: 24 Hours After Recovery (Evaluable Patient) - oral administration** | | | | | | |
|---|---|---|---|---|---|---|
| | Placebo (n = 62) | 03 µg/kg (n = 30) | 1 µg/kg (n = 57) | 3 µg/kg (n = 54) | 10 µg/kg (n = 49) | 30 µg/kg (n = 67) |
| % with CRp-value^{a} | 33% | 37% | 58% 0.013 | 52% 0.059 | 59% 0.009 | 53% 0.018 |
| % with CC pvalue^{a} | 33% | 37% | 58% 0.013 | 52% 0.059 | 57% 0.016 | 51% 0.038 |
| % no EE p-value^{a} | 40% | 50% | 67% 0.007 | 63% 0.019 | 71% 0.001 | 65% 0.008 |

| | | | | | | |
|---|---|---|---|---|---|---|
| complete response (CR), complete control (CC), and no emetic episodes (EE) for the six dose groups ^{a} p-value for treatment effet versus placebo | | | | | | |

### Onset and Duration of Antiemetic Activity

Within the first 4 hours of recovery, between 61% and 76% of the evaluable patients treated with palonosetron 1-30 µg/kg had a complete response, compared with 56% of placebo-treated patients (32/57). Only the 10-µg/kg dose was statistically significantly superior to placebo (p = 0.04). Over this same time period, statistically significantly more patients in the 3 and 10 µg/kg treatment groups were free from emetic episodes, compared with the placebo group (81% and 82% vs. 63%; p -< 0.043); whereas there was no difference among the .1.0- and 30-µg/kg and placebo treatment groups (72% and 69% vs. 63%).

| **Table 6: 4 Hours After Recovery (Evaluable Patients) - oral** | | | | | | |
|---|---|---|---|---|---|---|
| | Placebo (n = 62) | 0.1 µg (n = 47) | 0.3 µg/kg (n = 67) | 1 µg/kg (n = 62) | 3 µg/kg (n = 67) | 30 µg/kg (n = 67) |
| % with CR p-value^{a} | 56% | 60% | 61% 0.656 | 70% 0.149 | 76% 0.04 | 63% 0238 |
| % no EE p-value^{a} | 63% | 73% | 72% 0367 | 81% 0.043 | 82% 0.043 | 69% 0.307 |

| | | | | | | |
|---|---|---|---|---|---|---|
| ^{a} p-value for treatment effect versus placebo | | | | | | |

### Control of Nausea

Patients who received palonosetron 1, 3, 10, and 30 µg/kg reported statistically significantly less nausea over the 24-hour period following recovery than those who received placebo. Approximately half of patients (44.8%-55.8%) who received palonosetron 1-30 µg/kg reported no nausea, and 2.2%-7.7% of patients reported severe Nausea. By comparison, 10.5% of placebo treated patients (6/57) reported severe nausea and 31.6% (18157) reported no nausea (p-value vs. placebo <; 0.015). The palonosetron 0.3 µg/kg treatment group was intermediate to the placebo group and the other palonosetron treatment groups.

| **Table 7: Nausea During 24 Hours After Recovery - oral** | | | | | | |
|---|---|---|---|---|---|---|
| | Placebo (n = 62) | 0.3 µg/kg (n = 29) | 1 µg/kg (n = 53) | 3 µg/kg (n = 52) | 10 µg/kg (n = 45) | 30 µg/kg (n = 58) |
| No Nausea | 31.6% | 44.8% | 52.8% | 55.8% | 51.1% | 44.8% |
| Severe Nausea | 10.5% | 3.4% | 3.8% | 7.7% | 2.2% | 5.2% |
| Mean Score | 1.0 | 0.83 | 0.64 | 0.65 | 0.71 | 0.79 |
| p-value Placebo | | N/A | 0.012 | 0.015 | 0.012 | 0.007 |

### Rescue Antiemetics

Promethazine (Phenergan®) and droperidol (Inapsine®) were the most frequently used rescue antiemetics, administered to 15.1% and 13.1% of patients, respectively. Other antiemetics administered were metoclopramide (Reglan®, 8.8%) proclorperazine (Compazine®, 4.8%), scopolamine (Transderm Scop®, 2,0%), and trimethobenzamide (titan®, 03%). Four patients (1.1%) received ondansetron although the protocol stated that patients should not be rescued with 5-HT₃ receptor antagonists.

### EXAMPLE 3. INTRAVENOUS FORMULATION

Table 8 below presents a representative formulation of palonosetron formulated for intravenous administration.

| **Table 8: Representative IV Formulation** | |
|---|---|
| **Ingredient** | **Weight Parts (mg/ml)** |
| Palonosetron Hydrochloride | 0.05 |
| Mannitol | 41.5 |
| EDTA | 0.5 |
| Trisodium citrate | 3.7 |
| Citric acid | 1.56 |
| WFJ | 1.0 |
| sodium hydroxide solution and/or hydrochloric acid solution | pH 5.0 ± 0.5 |

### EXAMPLE 4. ORAL FORMULATION

Table 9 below presents a representative formulation of palonosetron formulated for oral administration.

| **Table 9: Representative Oral Formulation** | |
|---|---|
| **Ingredient** | **Weight Parts (mg/ml)** |
| Palonosetron Hydrochloride | 0.05 |
| Mannitol | 1.50 |
| EDTA | 0.5 |
| Trisodium citrate | 3.7 |
| Citric acid | 1.56 |
| WFJ | 1.0 |
| Sodium hydroxide solution and/or pH hydrochloric acid solution | 5.0 ± 0.5 |
| Flavoring | q.s. |

## Claims

1. Use of from 0.025 to 0.250 mg of a treatment-effective amount of palonosetron in the manufacture of a medicament for the treatment or prevention of post-operative nausea and vomiting (PONV) in a human, wherein said palonosetron is contained in a liquid formulation at a concentration ranging from 0.03 to 0.2 mg/ml, and wherein said palonosetron is administered in a window about 4 hours before the operation to about 4 hours after the operation.

2. The use of claim 1 wherein said medicament dose is used as a rescue medication.

3. The use of claim 1 wherein said medicament comprises 0.025, 0.05 or 0.075 mg of palonosetron.

4. The use of claim 1 wherein said concentration is 0.05 mg/ml palonosetron.

5. The use of claim 1 wherein said medicament dose comprises a single unit dose vial, which comprises 0.1 to 10 ml of solution.

6. The use of claim 1 wherein said medicament dose is an oral solution.

7. The use of claim 1 wherein said medicament dose is an intravenous solution.

8. The use of claim 6 or claim 7 wherein said medicament dose has a pH from 4.5 tb 5.5.

9. Use of claim 1 wherein said medicament is an oral/intravenous solution and has a pH of from 4.5 to 5.5 and further comprises a chelating agent, and/or a buffer.

10. The use of claim 9 wherein said medicament dose comprises 0.025, 0.05 or 0.075 mg of palonosetron.

11. The use of claim 9, wherein said palonosetron is present at a concentration of from 0.03 to 0.2 mg/ml.

12. The use of claim 9 wherein said medicament dose is used as a rescue medicament

13. The use of claim 9 wherein said medicament dose comprises a single unit dose vial comprising from 0.1 to 10.0 ml of solution.

14. The use of claim 9 wherein said medicament dose is an oral solution.

15. The use of claim 9 wherein said medicament dose is an intravenous solution.

16. The use of one of claims 1 or 9 wherein said PONV is delayed on-set PONV.

17. The use of one of claims 1 or 9 in the manufacture of a medicament dose for the treatment or prevention of PONV as a single unit dose vial comprising from 0.1 to 10.0 ml of solution over a period of even days
from the emesis inducing event.

18. The use af claim 1 or 9 in an amount of from 0.025 to 0.20 mg palonosatron.

## Patentansprüche

1. Verwendung von 0,025 bis 0,250 mg einer behandlungswirksamen Menge von Palonosetron für die Herstellung eines Arzneimittels zur Behandlung oder Vorbeugung von postoperativer Übelkeit und Erbrechen (*post-operative nausea and vomiting;* PONV) im Menschen, wobei das Palonosetron in einer flüssigen Formulierung enthalten ist mit einer Konzentration wischen 0,03 bis 0,2 mg/ml, und wobei das Palonosetron in einem Fenster von ungefähr 4 Stunden vor der Operation bis ungefähr 4 Stunden nach der Operation verabreicht wird

2. Verwendung gemäß Anspruch 1, wobei die Arzneimitteldosis als eine Rettungsmedikation verwendet wird

3. Verwendung gemäß Anspruch 1, wobei das Arzneimittel 0,025, 0,05 oder 0,075 mg von Palonosetron umfasst.

4. Verwendung gemäß Anspruch 1, wobei die Konzentration 0,05 mg/ml Palonosetron ist.

5. Verwendung gemäß Anspruch 1, wobei die Arzneimitteldosis eine einzelne Einheitsdosisampulle umfasst, die 0,1 bis 10 ml Losung umfasst.

6. Verwendung gemäß Anspruch 1, wobei die Arzneimitteldosis eine orale Lösung ist.

7. Verwendung gemäß Anspruch 1, wobei die Arzneimitteldosis eine intravenöse Lösung ist.

8. Verwendung gemäß Anspruch 6 oder Anspruch 7, wobei die Arzneimitteldosis einen pH-Wert von 4,5 bis 5,5 hat.

9. Verwendung gemäß Anspruch 1, wobei das Arzneimittel eine orale/intravenöse Losung ist und einen pH-Wert von 4,5 bis 5,5 hat und weiterhin einen Komplexbildner und/oder einen Puffer umfasst.

10. Verwendung gemäß Anspruch 9, wobei die Arzneimitteldosis 0,025, 0,05 oder 0,075 mg von Palonosetron umfasst.

11. Verwendung gemäß Anspruch 9, wobei das Palonosetron in einer Konzentration von 0,03 bis 0,2 mg/ml vorhanden ist.

12. Verwendung gemäß Anspruch 9, wobei die Arzneimitteldosis als eine Rettungsmedikation verwendet wird

13. Verwendung gemäß Anspruch 9, wobei die Arzneimitteldosis eine einzelne Einheitsdosisampulle umfasst, die von 0,1 bis 10,0 ml der Losung umfasst.

14. Verwendung gemäß Anspruch 9, wobei die Arzneimitteldosis eine orale Lösung ist

15. Verwendung gemäß Anspruch 1, wobei die Arzneimitteldosis eine intravenöse Losung ist.

16. Verwendung gemäß einem der Ansprüche 1 oder 9, wobei das PONV ein sich im Ausbruch verzögerndes PONV ist.

17. Verwendung gemäß einem der Ansprüche 1 oder 9 für die Herstellung einer Arzneimitteldosis zur Behandlung oder Vorbeugung von PONV als eine einzelne Einheitsdosisampulle, umfassend von 0,1 bis 10,0 ml der Lösung, uber einen Zeitraum von sieben Tagen ab dem einleitenden Ereignis für das Erbrechen.

18. Verwendung gemäß Anspruch 1 oder 9 in einer Menge von 0,025 bis 0,20 mg Palonosetron.

## Revendications

1. Utilisation de 0,025 à 0,250 mg d'une quantité thérapeutique efficace de palonosétron dans la fabrication d'un médicament destiné au traitement ou à la prévention des nausées et des vomissements postopératoires (PONV) chez un être humain, où ledit palonosétron est contenu dans une formulation liquide à une concentration située dans la plage de 0,03 à 0,2 mg/ml, et où ledit palonosétron est administré dans une fenêtre d'environ 4 heures avant l'opération jusqu'à environ 4 heures après l'opération.

2. Utilisation selon la revendication 1, où ladite dose médicamenteuse est utilisée en tant que médicament de secours.

3. Utilisation selon la revendication 1, où ledit médicament comprend 0,025, 0,05 ou 0,075 mg de palonosétron.

4. Utilisation selon la revendication 1, où ladite concentration est de 0,05 mg/ml de palonosétron.

5. Utilisation selon la revendication 1, où ladite dose médicamenteuse compose un seul flacon de dose unitaire qui comprend 0,1 à 10 ml de solution.

6. Utilisation selon la revendication 1, où ladite dose médicamenteuse est une solution orale.

7. Utilisation selon la revendication 1, où ladite dose médicamenteuse est une solution intraveineuse.

8. Utilisation selon la revendication 6 ou la revendication 7, où ladite dose médicamenteuse a un pH de 4,5 à 5,5.

9. Utilisation selon la revendication 1, où ledit médicament est une solution orale/intraveineuse et a un pH de 4,5 à 5,5 et comprend en outre un agent chélateur et/ou un tampon.

10. Utilisation selon la revendication 9, où ladite dose médicamenteuse comprend 0,025, 0,05 ou 0,075 mg de palonosétron.

11. Utilisation selon la revendication 9, où ledit palonosétron est présent à une concentration de 0,03 à 0,2 mg/ml.

12. Utilisation selon la revendication 9, où ladite dose médicamenteuse est utilisée en tant que médicament de secours.

13. Utilisation selon la revendication 9, où ladite dose médicamenteuse compose un seul flacon de dose unitaire qui comprend 0,1 à 10,0 ml de solution.

14. Utilisation selon la revendication 9, où ladite dose médicamenteuse est une solution orale.

15. Utilisation selon la revendication 9, où ladite dose médicamenteuse est une solution intraveineuse.

16. Utilisation selon l'une des revendications 1 ou 9, où lesdits PONV sont des PONV à installation retardée.

17. Utilisation selon l'une des revendications 1 ou 9, dans la fabrication d'une dose médicamenteuse destinée au traitement ou à la prévention des PONV sous la forme d'un seul flacon de dose unitaire comprenant de 0,1 à 10,0 ml de solution sur une période de sept jours à partir de l'événement induisant des vomissements.

18. Utilisation selon l'une des revendications 1 ou 9, dans une quantité de 0,025 à 0,20 mg de palonosétron.
